# EUROPEAN PATENT SPECIFICATION

(11) **EP 1 621 198 B1**
(45) Date of publication and mention of the grant of the patent: **23.05.2007**
(21) Application number: 05023971.4
(22) Date of filing: 29.01.2002
(51) Int. Cl.: A61K 31/496, A61K 45/06, A61P 25/18, A61P 25/28

(54) **Substituted carbostyril derivatives as 5-HT 1A receptor subtype agonists**
Substituierte Carbostyrilderivate als 5-HT1A-Subtyp Agonisten
Dérivés substitués de carbostyril comme agonistes du sous-type du récepteur 5-HT1A

(30) Priority: 29.01.2001 US 770210
(43) Date of publication of application: 01.02.2006
(62) Divisional of application: 02716434.2
(73) Proprietor: OTSUKA PHARMACEUTICAL CO., LTD., Chiyoda-ku Tokyo 101-8535 (JP)
(72) Inventor: Uwahodo, Yasufumi, Tokushima-shi, Tokushima 771-1151 (JP); Jordan, Shaun, Maryland, MD21704 (US); Kikuchi, Tetsuro, Tokushima-shi, Tokushima 771-0104 (JP); Tottori, Katsura, Itano-gun, Tokushima 771-1345 (JP); Hirose, Tsuyoshi, Tokushima-shi, Tokushima 770-0021 (JP)
(74) Representative: HOFFMANN EITLE

(56) References cited:
- EP-A- 0 367 141
- WO-A-99/38864
- US-A- 4 734 416
- US-A- 5 691 330
- LIEBERMAN JEFFREY A: "Atypical antipsychotic drugs as a first-line treatment of schizophrenia: A rationale and hypothesis." JOURNAL OF CLINICAL PSYCHIATRY, vol. 57, no. SUPPL. 11, 1996, pages 68-71, XP008005973 ISSN: 0160-6689
- MELTZER H Y ET AL: "SINGLE OR MULTIPLE RECEPTOR TARGETS: WHICH ARE BEST FOR ANTIPSYCHOTIC DRUGS" NEUROPSYCHOPHARMACOLOGY, ELSEVIER SCIENCE PUBLISHING, NEW YORK, NY, US, vol. 23, no. S02, August 2000 (2000-08), page S73, XP008029267 ISSN: 0893-133X
- INOUE T ET AL: "EFFECTS OF THE NOVEL ANTIPSYCHOTIC AGENT 7-ä4--(2,3-DICHLOROPHENYL)-1 PIPERAZINYL-1-PIPERAZINYLBUTYLOXYü-3,4-DIH YDRO-2(1H)-QUINOLINONE (OPC-14597) ON PROLACTIN RELEASE FROM THE RAT ANTERIOR PITUITARY GLAND" JOURNAL OF PHARMACOLOGY AND EXPERIMENTAL THERAPEUTICS, AMERICAN SOCIETY FOR PHARMACOLOGY AND, US, vol. 277, no. 1, April 1996 (1996-04), pages 137-143, XP008005978 ISSN: 0022-3565
- PRINSSEN ERIC P M ET AL: "Interactions between neuroleptics and 5-HT1A ligands in preclinical behavioral models for antipsychotic and extrapyramidal effects." PSYCHOPHARMACOLOGY, vol. 144, no. 1, May 1999 (1999-05), pages 20-29, XP002209298 ISSN: 0033-3158
- UWAHODO YASUFUMI ET AL: "Pharmacological profile of OPC-14597, a novel antipsychotic drug (2): Weak extrapyramidal side effects." JAPANESE JOURNAL OF PHARMACOLOGY, vol. 67, no. SUPPL. 1, 1995, page 144P, XP001237224 68th Annual Meeting of the Japanese Pharmacological Society;Nagoya, Japan; March 25-28, 1995 ISSN: 0021-5198
- GARCIA-NANYA M., APIQUIAN R., FRESAN A.: "Atypical antipsychotics: Review article [Los antipsycoticos atipicos: Una revision]" SALUD MENTAL, vol. 24, no. 5, October 2001 (2001-10), pages 37-43, XP008005976
- JORDAN S ET AL: "IN VIVO EFFECTS OF ARIPIPRAZOLE ON DOPAMINERGIC AND SEROTONERGIC FUNCTION IN RAT PREFRONTAL CORTEX AND STRIATUM" SOCIETY FOR NEUROSCIENCE ABSTRACTS, SOCIETY FOR NEUROSCIENCE, US, vol. 2, no. 27, 2001, page 2327,AN87503, XP008005982 ISSN: 0190-5295
- DATABASE BIOSIS [Online] BIOSCIENCES INFORMATION SERVICE, PHILADELPHIA, PA, US; 2001, SUMIYOSHI T ET AL: "Tandospirone, a serotonin-1A agonist, added to neuroleptic treatment enhances cognitive performance in schizophrenia" XP002364564 Database accession no. PREV200200022926 & SOCIETY FOR NEUROSCIENCE ABSTRACTS, vol. 27, no. 2, 2001, page 2586, 31ST ANNUAL MEETING OF THE SOCIETY FOR NEUROSCIENCE; SAN DIEGO, CALIFORNIA, USA; NOVEMBER 10-15, 2001 ISSN: 0190-5295

## Description

### BACKGROUND OF THE INVENTION

### FIELD OF THE INVENTION

The present invention relates to the use of specific carbostyril compounds for the production of a medicament for the treatment of specific disorders of the central nervous system associated with the 5-HT_{1A} receptor subtype.

### RELATED ART

U.S. Patent No. 5,006,528; European Patent No. 367,141 and Japanese Patent Kokai (Laid-open)7-304,740 (1995) contain the same chemical structural formula as the carbostyril derivatives in the present invention, and their pharmacological properties are beneficial drug treatments for schizophrenia.

Carbostyril compounds, as well as those disclosed in Japanese Patent Kokai (Laid-open)9-301,867 (1997) are useful for the treatment of anxiety.

The carbostyril derivatives disclosed in European Patent No. 226,441 have the genus of the carbostyril derivatives in the present invention, and they are useful for the treatment of hypoxia.

In addition to the above, the carbostyril derivatives disclosed in U.S. Patent No. 4,734,416; Canadian Patent No. 1,117,110; British Patent No. 2,017,701; German Patent Nos. 2,911,108, 1,912,105 and 2,953,723; Japanese Patent Kokai(Laid-open)Nos. 54-130,587 (1979), 55-127,371 (1980) and 62-149,664 (1987) have the genus of the carbostyril derivatives in the present invention, and they have antihistaminic activities and central nervous controlling activities.

It is reported that aripiprazole (7-{4-[4-(2,3-dichlorophenyl)-1-piperazinyl]butoxy}-3,4-dihydrocarbostyril, also known as OPC-14597, BMS-337,039 and OPS-31) binds with high affinity to dopamine D₂ receptors and with moderate affinity to dopamine D₃ and 5-HT₇ receptors (Masashi Sasa et al., CNS Drug Reviews, Vol. 3, No. 1, pp. 24-33).

Further, it is reported that aripiprazole possesses presynaptic dopaminergic autoreceptor agonistic activity, postsynaptic D₂ receptor antagonistic activity, and D₂ receptor partial agonistic activity (T. Kikuchi, K. Tottori, Y. Uwahodo, T. Hirose, T. Miwa, Y. Oshiro and S. Morita: J. Pharmacol. Exp. Ther., Vol. 274, pp. 329, (1995); T. Inoue, M. Domae, K. Yamada and T. Furukawa: J. Pharmacol. Exp. Ther., Vol. 277, pp. 137, (1996)).

However, it has not been reported that compounds in the present invention have agonistic activity at 5-HT_{1A} receptor subtype.

It has been reported that therapeutic interventions using 5-HT_{1A} receptor ligands may be useful drug treatments for alcohol abuse (Mark Kleven et al., European Journal of Pharmacology, Vol. 281, (1995) pp. 219-228).

It is also reported that 5-HT_{1A} agonist drugs may be useful for the treatment and/or prophylaxis of disorders associated with neuronal degeneration resulting from ischemic events in mammals (U.S. Patent No. 5,162,375).

It is also reported that 5-HT_{1A} receptor hypersensitivity could be the biological basis for the increased frequency of migraine attack in stressful and anxious conditions (Massimo Leone et al., Neuro Report, Vol. 9, pp. 2605-2608(1998)).

It has recently been reported that (-)-(R)-2-[4-[[(3,4-dihydro-2H-1-benzopyran-2-yl)methyl]amino]-butyl]-1,2-benzisothiazol-3(2H)-one 1,1-dioxide monohydrochrolide (BAY-3702), a 5-HT_{1A} receptor agonist, has neuroprotective, anxiolytic- and antidepressant-like effects in animal models (Jean De Vry et al., European Journal of Pharmacology, Vol. 357, (1998), pp. 1-8).

It is also reported that 5-HT_{1A} receptor agonists appear to be broad spectrum antiemetic agents (Mary C. Wolff et al., European Journal of Pharmacology, Vol. 340, (1997), pp. 217-220; AB Alfieri et al., British Journal of Cancer, (1995), Vol. 72, pp. 1013-1015; Mary C. Wolff et al., Pharmacology Biochemistry and Behavior, 1995, Vol. 52, No. 3, pp. 571-575; James B. Lucot, European Journal of Pharmacology, 1997, Vol. 253, pp. 53-60).

Serotonin plays a role in several neurological and psychiatric disorders, including Alzheimer's disease, depression, nausea and vomiting, eating disorders, and migraine. (See Rasmussen et al., "Chapter 1. Recent Progress in Serotonin 5HT_{1A} Receptor Modulators", in Annual Reports in Medicinal Chemistry, Vol. 30, Section I, pp. 1-9, 1995, Academic Press, Inc.). WO 00/16777 discloses that a 5HT_{1A} receptor agonist, buspirone is efficacious in treating a variety of symptoms associated with ADHD, and that combined use of a D2 receptor agonist and 5-HT_{1A} agonist provides effective treatments for ADHD and Parkinson's disease.

5HT_{1A} agonists are effective in the treatment of cognitive impairment in Alzheimer's disease, Parkinson's disease or senile dementia. US 5824680 discloses that a 5-HT_{1A} agonist, ipsapirone, is effective in treating Alzheimer's disease by improving memory. US 4687772 describes that a 5-HT_{1A} partial agonist, buspirone, is useful for improving short term memory in patients in need of treatment. WO 93/04681 discloses that use of 5-HT_{1A} partial agonists have been used for the treatment or prevention of cognitive disorders associated with Alzheimer's disease, Parkinson's disease or senile dementia.

5HT_{1A} agonists are also effective in the treatment of depression. US 4771053 describes that a 5-HT_{1A} receptor partial agonist, gepirone, is useful in alleviation of certain primary depressive disorders, such as severe depression; endogenous depression, major depression with melancholia, and atypical depression. WO 01/52855 discloses that the combined use of the 5-HT_{1A} receptor partial agonist gepirone with an antidepressant can effectively treat depression.

The 5-HT_{1A} receptor partial agonist buspirone alleviates motor disorders such as neuroleptic induced parkinsonism and extrapyramidal symptoms. These observations are disclosed in US 4438119. Furthermore 5-HT_{1A} agonists reverse neuroleptic-induced catalepsy in rodents, which mimic movement impairments observed in Parkinson's disease (Mark J. Millan, Journal of Pharmacology and Experimental Therapeutics, 2000, Vol. 295, p853-861). Thus, aripiprazole can be used to manage psychosis in geriatric patients, Alzheimer's disease, Parkinson's disease or senile dementia, since it possesses potent, partial agonistic activities at D₂ and 5-HT_{1A} receptors. In addition, these patients might not experience extrapyramidal symptoms due to this property of aripiprazole.

Heretofore, schizophrenia is understood to be caused by hyperactivity in the brain dopaminergic system. For this reason, some drugs were developed with strong dopaminergic receptor blocking activity. These typical antipsychotic drugs are effective in the treatments for the positive symptoms of schizophrenia, which include hallucinations, delusions and the like. During the last decade, a variety of atypical antipsychotic drugs have been developed, which include clozapine, risperidone, olanzapine, quetiapine. These drugs have less extrapyramidal side effects, and have other activities in addition to their DA-receptor blocking activities. In contrast to typical antipsychotic drugs, such as chlorpromazine, haloperidol, etc., it is reported that atypical antipsychotic drugs are more effective against the negative symptoms and cognitive impairments associated with schizophrenia than typical antipsychotic drugs, and atypical antipsychotic drugs also have less extrapyramidal side effects (S. Miyamoto, G. E. Duncan, R. B. Mailman and J. A. Lieberman: Current Opinion in CPNS Investigational Drugs, Vol. 2, pp. 25, (2000)). However, even though atypical antipsychotic drugs provide a suitable pharmacotherapy for schizophrenia, certain patients are resistant to the antipsychotic therapies of these drugs. These patients may either not respond or may become refractory (i.e. may feel more anxious, depressed or cognitive dysfunction) in response to antipsychotic therapy. These treatment-resistant patients pose a problem for how a physician may provide an appropriate therapy.

At present, a number of treatment-resistant and treatment-refractory schizophrenic patients display symptoms that do not respond adequately to a variety of known effective classes and doses of typical or atypical antipsychotic drugs. Furthermore, these patients may also be inveterate schizophrenia or chronic schizophrenics who are often repeatedly admitted to and discharged from hospitals (R. R. Conely and R. W. Buchanan: Schizophr. Bull., Vol. 23, pp. 663, (1997)).

Symptoms of patients corresponding to treatment-resistant and treatment-refractory schizophrenics involve not only the positive symptoms, but also the negative symptoms and emotional disorders, as well as cognitive impairments (i.e., cognitive dysfunction or cognitive disturbances) (K. Akiyama and S. Watanabe: Jpn. J. Clin. Psychopharmacol., Vol. 3, pp. 423, (2000)).

Cognitive impairment exists separately from the psychic symptoms in a schizophrenic individual. Thus, medical treatment is therefore quite important, because the cognitive impairment may disturb the socially adaptable behavior of these individuals (C. Hagger, P. Buckley, J. T. Kenny, L. Friedman, D. Ubogy and H. Y. Meltzer: Biol. Psychiatry, Vol. 34, pp. 702, (1993); T. Sharma and D. Mockler: J. Clin. Psychopharmacol., Vol. 18, (Suppl. 1), pp. 128, (1998)).

At present, clozapine is an antipsychotic drug that is effective against treatment-resistant schizophrenia. Clozapine (marketed under the name of Clozaril) was approved in 1990 by FDA for the treatment and management of severely ill schizophrenics who failed to respond adequately to standard antipsychotic therapy (M. W. Jann: Pharmacotherapy, Vol. 11, pp. 179, (1991)). Clozapine has been reported to be effective against cognitive impairments in treatment-resistant schizophrenics (C. Hagger, P. Buckley, J. T. Kenny, L.

Friedman, D. Ubogy and H. Y. Meltzer: Biol. Psychiatry, Vol. 34, pp. 702, (1993); M. A. Lee, P. A. Thompson and H. Y. Meltzer: J. Clin. Psychiatry, Vol. 55 (Suppl. B), pp. 82, (1994); D. E. M. Fujii, I. Ahmed, M. Jokumsen and J. M. Compton: J. Neuropsychiatry Clin. Neurosci., Vol. 9, pp. 240, (1997)). For example, it is reported that clozapine improves cognitive impairments in attention, response time, fluent-speech, etc. in treatment-resistant schizophrenics (M. A. Lee, P. A. Thompson and H. Y. Meltzer: J. Clin. Psychiatry, Vol. 55 (Suppl. B), pp. 82, (1994)). It has been also reported that clozapine provides effective improvements in cognitive impairments in an objective evaluation scale of the Wechsler Adult Intelligence Scale-Revised Full Scale (D. E. M. Fujii, I. Ahmed, M. Jokumsen and J. M. Compton: J. Neuropsychiatry Clin. Neurosci., Vol. 9, pp. 240, (1997)) .

The 5-HT_{1A} receptor has been demonstrated to play a role in the therapeutic efficacy of clozapine against treatment-resistant schizophrenia and cognitive impairments. This relationship was revealed by a binding experiment using human the 5-HT_{1A} receptors (S. L. Mason and G. P. Reynolds: Eur. J. Pharmacol., Vol. 221, pp. 397, (1992)). Further, in accordance with progress in molecular pharmacology, it is clearly understood that 5-HT_{1A} receptor agonistic activity or 5-HT_{1A} receptor partial agonistic activity plays an important role in treatment-resistant schizophrenia and cognitive impairments (A. Newman-Tancredi, C. Chaput, L. Verriele and M. J. Millan: Neuropharmacology, Vol. 35, pp. 119, (1996)). Additionally, it was reported that the number of 5-HT_{1A} receptor is increased in the prefrontal cortex of chronic schizophrenics who were classified treatment-resistant. This observation was explained by a compensatory process where by the manifestation of severe symptoms of chronic schizophrenia are a result of impaired neuronal function mediated by hypofunctional 5-HT_{1A} receptors (T. Hashimoto, N. Kitamura, Y. Kajimoto, Y. Shirai, O. Shirakawa, T. Mita, N. Nishino and C. Tanaka: Psychopharmacology, Vol. 112, pp. S35, (1993)). Therefore, a lowering in neuronal transmission mediated through 5-HT_{1A} receptors is expected in treatment-resistant schizophrenics. Thus the clinical efficacy of clozapine may be related to its partial agonist efficacy at the 5-HT_{1A} receptors (A. Newman-Tancredi, C. Chaput, L. Verriele and M. J. Millan: Neuropharmacology, Vol. 35, pp. 119, (1996)). 5-HT_{1A} receptor agonistic activity may be related to the clinical effects of clozapine, and this hypothesis is supported by a positron emission tomography study in primates which showed that clozapine interacts with brain 5-HT_{1A} receptors at a therapeutically effective dose (Y. H. Chou, C. Halldin and L. Farde: Int. J. Neuropsycho-pharmacol., Vol. 4 (Suppl. 3), pp. S130, (2000)). Furthermore tandospirone, which is known as a selective 5-HT_{1A} receptor agonist, improved cognitive impairments in chronic schizophrenic patients (T. Sumiyoshi, M. Matsui, I. Yamashita, S. Nohara, T. Uehara, M. Kurachi and H. Y. Meltzer: J. Clin. Pharmacol., Vol. 20, pp. 386, (2000)). While, in animal tests, all reports do not always suggest that 5-HT_{1A} receptor agonist activity may be related to cognitive impairment, however, 8-OH-DPAT (8-hydroxy-2-(di-n-propylamino)tetralin), which is known as a selective 5-HT_{1A} receptor agonist, improves learning and memory impairments induced by scopolamine known as a muscarinic receptor antagonist, suggesting a relationship between 5-HT_{1A} receptor agonistic activity and improvements in cognitive impairments (M. Carli, P. Bonalumi, R. Samanin: Eur. J. Neurosci., Vol. 10, pp. 221, (1998); A. Meneses and E. Hong: Neurobiol. Learn. Mem., Vol. 71, pp. 207, (1999)).

Atypical antipsychotic drugs, such as risperidone and olanzapine, were marketed after clozapine, and it is reported that these drugs improve treatment-resistant schizophrenia or cognitive impairments in treatment-resistant schizophrenics (M. F. Green, B. D. Marshall, Jr., W. C. Wirshing, D. Ames, S. R. Marder, S. McGurck, R. S. Kern and J. Mintz: Am. J. Psychiatry, Vol. 154, pp. 799, (1997); G. Bondolifi, H. Dufour, M. Patris, J. P. May, U. Billeter, C. B. Eap and P. Baumann, on behalf of the risperidone Study Group: Am. J. Psychiatry, Vol. 155, pp. 499, (1998); A. Breier, S. H. Hamilton: Biol. Psychiatry, Vol. 45, pp. 403, (1999))

In contrast to reports that clozapine was moderately effective against treatment-resistant schizophrenia, risperidone and olanzapine were not consistently superior to typical antipsychotic drugs in their effectiveness against treatment-resistant schizophrenia. Thus, risperidone and olanzapine bind with lower affinity to human 5-HT_{1A} receptors (S. Miyamoto, G. E. Duncan, R. B. Mailman and J. A. Lieberman: Current Opinion in CPNS Investigational Drugs, Vol. 2, pp. 25, (2000)), and as such these drugs can not clearly perform activities through human 5-HT_{1A} receptors at clinical effective doses.

Therefore, at present, it is understood that clozapine is effective against treatment-resistant schizophrenia (D. W. Bradford, M. H. Chakos, B. B. Sheitman, J. A. Lieberman: Psychiatry Annals, Vol. 28, pp. 618, (1998); A. Inagaki: Jpn. J. Clin. Psycho-pharmacol., Vol. 3, pp. 787, (2000)).

As explained above, 5-HT_{1A} receptor agonistic activity is important for improving treatment-resistant schizophrenia or cognitive impairment caused by treatment-resistant schizophrenia. Clozapine is effective against treatment-resistant schizophrenia, however, its use is limited due to its severe side-effect of producing agranulocytosis which requires patients to undergo periodical blood tests. Under these circumstances, the development of a safe anti-psychotic drug with potent, full or partial agonist activity at 5-HT_{1A} receptors is earnestly desired.

The carbostyril compound in the present invention binds with high affinity and displays a potent, partial agonist activity at the 5-HT_{1A} receptors and it has higher intrinsic activity (about 68%) as compared with that of clozapine. Therefore, the compound in the present invention has a 5-HT_{1A} receptor agonistic activity that is more potent than the agonistic activity of clozapine. Thus, the present carbostyril compound may represent a more potent and highly safe drug for curing cognitive impairments caused by treatment-resistant schizophrenia, cognitive impairments caused by inveterate schizophrenia and cognitive impairments caused by chronic schizophrenia, as compared with other currently available pharmacotherapeutic treatments. That is, the compound in the present invention may prove to be a potent and safer drug therapy for cognitive impairments caused by treatment-resistant schizophrenia, cognitive impairments caused by inveterate schizophrenia, or cognitive impairments caused by chronic schizophrenia, which fail to respond to currently available antipsychotic drugs selected from chlorpromazine, haloperidol, sulpiride, fluphenazine, perphenazine, thioridazine, pimozide, zotepine, risperidone, olanzapine, quetiapine and amisulpride.

In particular, the carbostyril compound in the present invention may be a potent and highly safe drug therapy against cognitive impairments caused by treatment-resistant schizophrenia, cognitive impairments caused by inveterate schizophrenia or cognitive impairments caused by chronic schizophrenia, which fail to respond to both of 1 to 3 typical antipsychotic drugs selected from the group consisting of chlorpromazine, haloperidol and perphenazine, and one atypical antipsychotic drug selected from the group consisting of risperidone, olanzapine, quetiapine and amisulpride.

Moreover, the compound in the present invention may be a potent and highly safe drug therapy against cognitive impairments caused by treatment-resistant schizophrenia, cognitive impairment caused by inveterate schizophrenia or cognitive impairment caused by chronic schizophrenia, which fail to respond to both of 2 typical antipsychotic drugs selected from the group consisting of chlorpromazine, haloperidol and perphenazine, and one atypical antipsychotic drug selected from the group consisting of risperidone, olanzapine, quetiapine and amisulpride.

Moreover, the compound in the present invention may be a potent and highly safe drug therapy against cognitive impairments caused by treatment-resistant schizophrenia, cognitive impairments caused by inveterate schizophrenia or cognitive impairments caused by chronic schizophrenia, which fail to respond to both of 1 to 2 typical antipsychotic drugs selected from the group consisting of chlorpromazine and haloperidol, and one atypical antipsychotic drug selected from the group consisting of risperidone, olanzapine, quetiapine and amisulpride.

Moreover, the compound in the present invention may be a potent and highly safe drug therapy against cognitive impairments caused by treatment-resistant schizophrenia, cognitive impairment caused by inveterate schizophrenia or cognitive impairment caused by chronic schizophrenia, which fail to respond to both of 2 typical antipsychotic drugs selected from the group consisting of chlorpromazine and haloperidol, and one atypical antipsychotic drug selected from the group consisting of risperidone, olanzapine, quetiapine and amisulpride.

### SUMMARY OF THE INVENTION

According to the present invention there is provided the use of a carbostyril compound of the formula (1) according to claim 1 for the manufacture of a medicament for treating a patient suffering from a disorder of the central nervous system associated with the 5-HT_{1A} receptor subtype according to claim 1.

### DETAILED DESCRIPTION OF THE INVENTION

As the 5-HT_{1A} receptor subtype agonist compound for use in accordance with the present invention, carbostyril derivatives represented by the following formula (1) are used: 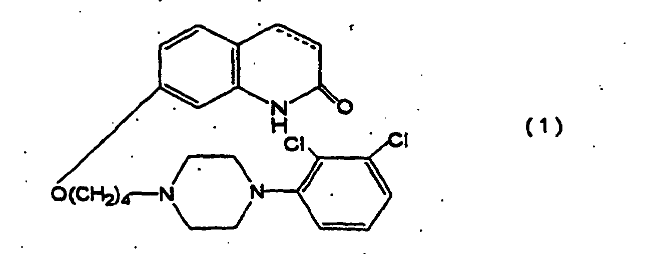 wherein the carbon-carbon bond between 3- and 4- positions in the carbostyril skeleton is a single or a double bond.

The compounds of the forgoing general formula (1) are known compounds, which are disclosed in publication such as U.S. Pat. No. 5,006,528 or which can be readily prepared by the processes described in the above publication.

The carbostyril derivative represented by the formula (1) in the present invention can easily be converted into its acid-addition salt by reacting it with a pharmaceutically acceptable acid. Examples of such acid include inorganic acids, such as hydrochloric acid, sulfuric acid, phosphoric acid and hydrobromic acid organic acids, such as oxalic acid, maleic acid, fumaric acid, malic acid, tartaric acid, citric acid and benzoic acid.

The solvent of solvates is a solvent conventionally used in recrystallization. Examples of solvates include hemihydrates, hydrates, and alcoholates, such as ethanolates, methanolates and isopropanolates.

The desired compounds, prepared by the reactions mentioned above, can easily be isolated and purified by usual separation procedures such as solvent extraction, dilution, recrystallization, column chromatography and preparative thin layer chromatography.

The potent, partial 5-HT_{1A} receptor agonist in the present invention is useful for disorders of the central nervous system associated with the 5-HT_{1A} receptor subtype selected from ; cognitive impairments caused by treatment-resistant schizophrenia, by inveterate schizophrenia or by chronic schizophrenia.

Compounds of the present invention may be suitably prepared into pharmaceutically acceptable formulations (see U.S. Patent No. 5,006,528, European Patent No. 367,141 and Japanese Kokai (Laid-open) 7-304,740 (1995), and Japanese Patent Application No. 2000-194976).

The dosage of these pharmaceutical preparations may be selected appropriately depending on the way of administration, the patient's age, sex and other factors, severity of the disease and other factors. Generally, however, the daily dose of the active ingredient compound is preferably within the range of 0.0001 to 50 mg per kilogram of body weight. It is desirable that the active ingredient compound be contained in each unit dosage form in an amount of 0.001 to 1,000 mg, particularly 0.01 to 100 mg, more particularly 0.1 to 50 mg, yet more particularly 1 mg to 20 mg.

### Pharmacological tests

### 1. MATERIALS AND METHODS

### 1.1 Test Compound

7-{4-[4-(2,3-Dichlorophenyl)-1-piperazinyl]-butoxy}-3,4-dihydrocarbostyril (aripiprazole) was used as test compound.

### 1.2 Reference Compounds

Serotonin (5-HT) and WAY-100635 (N-[2-[4-(2-methoxyphenyl)-1-piperazinyl]ethyl]-N-(2-pyridimyl)-cyclohexanecarboxamide, a 5-HT_{1A} receptor antagonist, manufactured by RBI (Natick, MA) were used as reference compounds.

### 1.3 Vehicle

Dimethyl sulfoxide (DMSO) manufactured by Sigma Chemical Co. (St. Louis, MO) was used as vehicle.

### 1.4 Preparation of Test and Reference Compounds

Test compound was dissolved in 100% dimethyl sulfoxide (DMSO) to yield 100 µm stock solutions (final concentration of DMSO in all tubes containing test compound was 1%, v/v). All other reference compounds were prepared by the same method using double-distilled water rather than DMSO.

### 1.5 Experimental Procedure for the [³⁵S]GTP_{γ}S Binding Assay

Test and reference compounds were studied in triplicate at 10 different concentrations (0.01, 0.1, 1, 5, 10, 50, 100, 1000, 10000 and 50000 nM) for their effects upon basal [³⁵S]GTP_{γ}S binding to h5-HT_{1A} CHO cell membranes. Reactions were performed in 5 ml glass test tubes containing 8 µl of test/reference drug mixed with 792 µl of buffer (25 mM Tris HC1, 50 mM NaCl, 5 mM MgCl₂, 0.1 mM EGTA, pH = 7.4) containing GDP (1 µM), [³⁵S ] GTP_{γ}S (0.1 nM) and h5-HT_{1A} CHO cell membranes (10 µg protein/reaction; NEN Life Science Products, Boston, MA; catalog # CRM035, lot # 501-60024, GenBank # X13556). Reactions proceeded for 60 min at room temperature and were terminated by rapid filtration through Whatman GF/B filter paper, using a Brandel harvester and 4x3 ml ice-cold buffer washes. ³⁵S radioactivity bound to the filter paper was measured using liquid scintillation counting (1272 Clinigamma, LKB/Wallach).

### 1.6 Experimental Procedure to Determine the Binding Affinity of Test compound (aripiprazole) at the h5-HT_{1A} Receptor

Test compound was studied in triplicate at 10 different concentrations (0.01, 0.1, 1, 10, 50, 100, 500, 1000, 5000 and 10000 nM) to determine its displacement of [³H]8-OH-DPAT (1 nM; NEN Life Sciences; catalog # NET 929, lot # 3406035, Specific Activity = 124.9 Ci/mmol) binding to h5-HT_{1A} receptors in CHO cell membranes (15 - 20 µg protein; NEN Life Science Products, catalog # CRM035, lot # 501-60024). Membranes (396 µl) were incubated in 5 ml glass tubes containing [³H]8-OH-DPAT (396 µl), test compound or vehicle (8 µl) and buffer A (50 mM Tris.HCl, 10 mM MgSO₄, 0.5 mM EDTA, 0.1% (w/v) ascorbic acid, pH = 7.4). All assays proceeded for 60 min at room temperature and were terminated by rapid filtration through Whatman GF/B filter paper (presoaked in buffer B; 50 mM Tris.HCl, pH = 7.4), using a Brandel harvester and 4x1 ml ice-cold washes with buffer B. Non-specific binding was determined in the presence of 10 µM (+)8-OH-DPAT.

### 1.7 Parameters Determined

Serotonin (5-HT) is a full 5-HT_{1A} receptor agonist which stimulates increases in basal [³⁵S]GTP_{γ}S binding to h5-HT_{1A} receptors in recombinant CHO cell membranes. Test compound was studied at 10 concentrations to determine their effects upon basal [³⁵S]GTP_{γ}S binding relative to that produced by 10 µM 5-HT. The relative potency (EC₅₀, 95% confidence interval) and intrinsic agonist activity (% of Eₘₐₓ for 10 µM 5-HT) was calculated for each compound by computerized non-linear regression analysis of complete concentration-effect data. The binding affinity of test compound at the h5-HT_{1A} receptor was determined by its ability to prevent [³H] 8-OH-DPAT binding to CHO cell membranes that express this receptor. Non-linear regression analysis of the competition binding data was used to calculate an inhibition constant (IC₅₀, 95% confidence interval), which is the concentration of test compound that occupies half of the h5-HT_{1A} sites specifically bound by [³H] 8-OH-DPAT. The affinity of h5-HT_{1A} receptors for test compound (Ki, 95% confidence interval) was calculated by the equation, Ki = (IC₅₀)/(1+([[³H]8-OH-DPAT]/Kd), where the Kd for [³H]8-OH-DPAT at h5-HT_{1A} = 0.69 nM (NEN Life Sciences). All estimates of drug binding affinity, potency and intrinsic efficacy at the h5-HT_{1A} receptor were calculated using GraphPad Prism version 3.00 for Windows (GraphPad Software, San Diego, CA).

### 2. RESULTS

Test compound and 5-HT produced concentration-dependent increases above basal [^{3S}S]GTP_{γ}S binding. 1% DMSO tested alone had no effect upon basal or drug-induced [³⁵S] GTP_{γ}S binding.

Test compound (EC₅₀ = 2.12 nM), 5-HT (EC₅₀ = 3.67 nM), potently stimulated basal [³⁵S]GTP_{γ}S binding. Potency and intrinsic agonist efficacy estimates were derived by non-linear regression analysis with correlation coefficients (r²)>0.98 in each case (Table 1). Test compound exerted partial agonist efficacies in the 65 - 70% range. WAY-100635 produced no significant change (unpaired Student's t-test) in basal [³⁵S]GTP_{γ}S binding at all concentrations tested (Table 1). WAY-100635 did, however, completely inhibit the effects of 5-HT and test compound upon [³⁵S]GTP_{γ}S binding to h5-HT_{1A} receptors in CHO cell membranes (Table 2). Tables 1 and 2 are shown below.

Test compound demonstrated high affinity binding to h5-HT_{1A} receptors in CHO cell membranes (IC₅₀ = 4.03 nM, 95% confidence interval = 2.67 to 6.08 nM; Ki = 1.65 nM, 95% confidence interval = 1.09 to 2.48 nM).

**Table 1 Potency (EC₅₀) and Intrinsic Agonist Efficacy (Eₘₐₓ) of Test compound and Reference Drugs in a h5-HT_{1A} [³⁵S] GTP_{γ}S CHO-cell Membrane Binding Assay.**

| Drug | EC₅₀, nM (95% Confidence Interval) | Eₘₐₓ (% ± SEM) | Goodness of Fit (r²) |
|---|---|---|---|
| Test Compound | 2.12 (0.87 to 5.16) | 68.13 ± 3.16 | 0.986 |
| 5-HT | 3.67 (1.56 to 8.63) | 98.35 ± 4.47 | 0.986 |
| WAY-100635 | - | - | - |

**Table 2 Inhibitory Potency (IC₅₀) of WAY-100635 versus 1 µM Concentration of 5-HT and Test compound in a h5-HT_{1A} [³⁵S] GTP_{γ}S CHO-cell Membrane Binding Assay.**

| Drug Combination | WAY-100635 Inhibition Potency, IC₅₀,nM (95% Confidence Interval) | Goodness of Fit (r²) |
|---|---|---|
| 5-HT + WAY-100635 | 217.1 (127..4 to 369.7) | 0.988 |
| Test compound + WAY-100635 | 392.2 (224.1 to 686.2) | 0.989 |

## Claims

1. Use of a carbostyril compound of the formula (1): 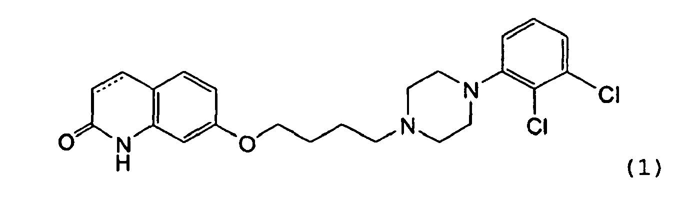 wherein the dotted line represents a single or a double bond, or a pharmaceutically acceptable salt or solvate thereof, for the production of a medicament for the treatment of disorders of the central nervous system associated with 5-HT_{1A} receptor subtype, selected from cognitive impairment caused by treatment-resistant schizophrenia, cognitive impairment caused by inveterate schizophrenia, or cognitive impairment caused by chronic schizophrenia.

2. The use of Claim 1, wherein the disorder fails to respond to antipsychotic drugs selected from chlorpromazine, haloperidol, sulpiride, fluphenazine, perphenazine, thioridazine, pimozide, zotepine, risperidone, olanzapine, quetiapine and amisulpride.

3. The use of Claim 2, wherein the disorder fails to respond to
(i) 1-3 typical antipsychotic drugs selected from chlorpromazine, haloperidol and perphenazine, and
(ii) one atypical antipsychotic drug selected from risperidone, olanzapine, quetiapine, and amisulpride.

4. The use of Claim 3, wherein the disorder fails to respond to two of the typical antipsychotic drugs.

5. The use of Claim 3, wherein the disorder fails to respond to chlorpromazine and/or haloperidol.

6. The use of Claim 5, wherein the disorder fails to respond to chlorpromazine and haloperidol.

7. The use of any of claims 1-6, wherein the carbostyril compound is 7-{4-[4-(2,3-dichlorophenyl)-1-piperaziny]butoxyl}-3,4-dihydrocarbostyril.

## Patentansprüche

1. Verwendung einer Carbostyrylverbindung der Formel (1): 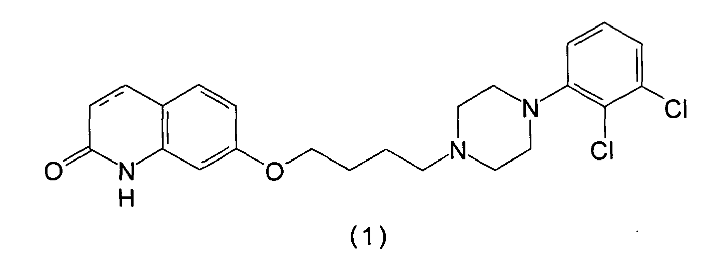 worin die gestrichelte Linie eine Einfach- oder Doppelbindung darstellt, oder eines pharmazeutisch akzeptablen Salzes oder Solvats hiervon, zur Herstellung eines Medikaments zur Behandlung von Störungen des zentralen Nervensystems, die mit dem 5-HT_{1A}-Rezeptor-Subtyp assoziiert sind, ausgewählt aus kognitiver Beeinträchtigung, die durch behandlungsresistente Schizophrenie verursacht wird, kognitiver Beeinträchtigung, die durch unverbesserliche Schizophrenie verursacht wird, oder kognitiver Beeinträchtigung, die durch chronische Schizophrenie verursacht wird.

2. Verwendung gemäss Anspruch 1, worin die Störung nicht auf antipsychotische Arzneimittel anspricht, die aus Chlorpromazin, Haloperidol, Sulpirid, Fluphenazin, Perphenazin, Thioridazin, Pimozid, Zotepin, Risperidon, Olanzapin, Quetiapin und Amisulprid ausgewählt sind.

3. Verwendung gemäss Anspruch 2, worin die Störung nicht auf
(i) 1-3 typische, antipsychotische Arzneimittel, die aus Chlorpromazin, Haloperidol und Perphenazin ausgewählt sind, und
(ii) ein atypisches antipsychotisches Arzneimittel, das aus Risperidon, Olanzapin, Quetiapin und Amisulprid ausgewählt ist,
anspricht.

4. Verwendung gemäss Anspruch 3, worin die Störung nicht auf zwei der typischen antipsychotischen Arzneimittel anspricht.

5. Verwendung gemäss Anspruch 3, worin die Störung nicht auf Chlorpromazin und/oder Haloperidol anspricht.

6. Verwendung gemäss Anspruch 5, worin die Störung nicht auf Chlorpromazin und Haloperidol anspricht.

7. Verwendung gemäss irgendeinem der Ansprüche 1 bis 6, worin die Carbostyrylverbindung
7-{4-[4-(2,3-Dichlorphenyl)-1-piperazinyl]butoxyl}-3,4-dihydrocarbostyryl ist.

## Revendications

1. Utilisation d'un composé carbostyrile de formule (1) : 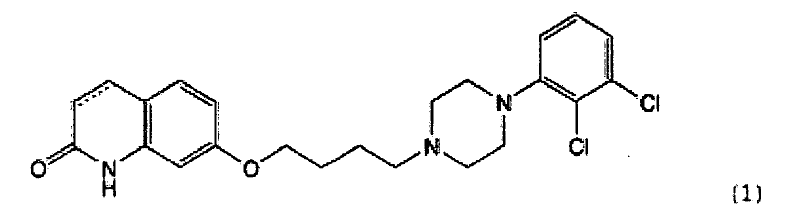 dans lequel la ligne pointillée représente une simple ou une double liaison, ou l'un de ses sels ou solvats pharmaceutiquement acceptables, pour la production d'un médicament pour le traitement de troubles du système nerveux central associés au sous-type de récepteur 5-HT_{1A}, choisis parmi un trouble cognitif provoqué par une schizophrénie résistant au traitement, un trouble cognitif provoqué par une schizophrénie invétérée, ou un trouble cognitif provoqué par une schizophrénie chronique.

2. Utilisation selon la revendication 1, dans laquelle le trouble ne répond pas aux médicaments antipsychotiques choisis parmi la chlorpromazine, l'halopéridol, le sulpiride, la fluphénazine, la perphénazine, la thioridazine, le pimozide, la zotépine, la rispéridone, l'olanzapine, la quétiapine et l'amisulpride.

3. Utilisation selon la revendication 2, dans laquelle le trouble ne répond pas à
(i) 1 à 3 médicaments antipsychotiques typiques choisis parmi la chlorpromazine, l'halopéridol et la perphénazine, et
(ii) un médicament antipsychotique atypique choisi parmi la rispéridone, l'olanzapine, la quétiapine et l'amisulpride.

4. Utilisation selon la revendication 3, dans laquelle le trouble ne répond pas à deux des médicaments antipsychotiques typiques.

5. Utilisation selon la revendication 3, dans laquelle le trouble ne répond pas à la chlorpromazine et/ou l'halopéridol.

6. Utilisation selon la revendication 5, dans laquelle le trouble ne répond pas à la chlorpromazine et l'halopéridol.

7. Utilisation selon l'une quelconque des revendications 1 à 6, dans laquelle le composé carbostyrile est le 7-{4-[4-(2,3-dichlorophényl)-1-pipérazinyl]butoxyl}-3,4-dihydrocarbostyrile.
